(19) Europäisches Patentamt
European Patent Office
Office européen des brevets

(11) Veröffentlichungsnummer: **0 179 021**
**A2**

(12) # EUROPÄISCHE PATENTANMELDUNG

(21) Anmeldenummer: 85810467.2

(22) Anmeldetag: 14.10.85

(51) Int. Cl.⁴: C 07 C 127/22
C 07 C 93/14, C 07 C 119/048
C 07 C 149/437, A 01 N 47/34

(30) Priorität: 18.10.84 CH 4993/84
08.11.84 CH 5361/84
14.05.85 CH 2048/85

(43) Veröffentlichungstag der Anmeldung:
23.04.86 Patentblatt 86/17

(84) Benannte Vertragsstaaten:
AT BE CH DE FR IT LI NL

(71) Anmelder: CIBA-GEIGY AG
Klybeckstrasse 141
CH-4002 Basel(CH)

(72) Erfinder: Drabek, Jozef, Dr.
Benkenstrasse 12
CH-4104 Oberwil(CH)

(72) Erfinder: Böger, Manfred
Wilhelm Glockstrasse 14
D-7858 Weil am Rhein 5(DE)

(54) **Benzoylphenylharnstoffe.**

(57) Neue substituierte N-Benzoyl-N'-2, 5-dichlor-4-halogenalkoxy-phenylharn-stoffe der Formel

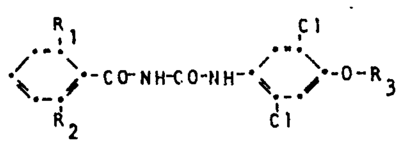

(I),

worin $R_1$ Wasserstoff, Fluor, Chlor, Methyl, Methoxy oder Methylthio, $R_2$ Fluor, Chlor, Methyl, Methoxy oder Methylthio und $R_3$ $C_1$–$C_7$-Halogenalkyl mit 1 bis 15 Halogenatomen oder $C_3$–$C_7$-Halogencycloalkyl mit 1 bis 13 Halogenatomen bedeuten, mit der Massgabe, dass $R_3$ nicht die Bedeutung $-CF_2-CHF-CF_3$ hat. Verfahren und Zwischenprodukte zur Herstellung dieser Verbindungen sowie diese enthaltende Mittel zur Bekämpfung von Insekten and Vertretern der Ordnung Akarina, speziell von pflanzenschädigenden Insekten, insbesondere als Ovizide.

EP 0 179 021 A2

CIBA-GEIGY AG 5-15117/144/B
Basel (Schweiz)

Benzoylphenylharnstoffe

Die vorliegende Erfindung betrifft neue substituierte N-Benzoyl-N'-2,5-dichlor-4-halogenalkoxy-phenylharnstoffe, Verfahren und Zwischenprodukte zu ihrer Herstellung und ihre Verwendung in der Schädlingsbekämpfung.

Die erfindungsgemässen Verbindungen haben die Formel I

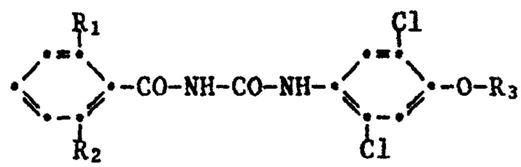 (I),

worin
$R_1$ Wasserstoff, Fluor, Chlor, Methyl, Methoxy oder Methylthio, $R_2$ Fluor, Chlor, Methyl, Methoxy oder Methylthio und $R_3$ $C_1$-$C_7$-Halogenalkyl mit 1 bis 15 Halogenatomen oder $C_3$-$C_7$-Halogencycloalkyl mit 1 bis 13 Halogenatomen bedeuten, mit der Massgabe, dass $R_3$ nicht die Bedeutung $-CF_2-CHF-CF_3$ hat. Insbesondere kann $R_3$ mit 1 bis 7 Halogenatomen substituiertes $C_1$-$C_3$-Alkyl bedeuten.

Wegen ihrer biologischen Wirkung bevorzugt sind diejenigen Verbindungen der Formel I, worin
$R_2$ Methyl, Methoxy oder Methylthio bedeutet, wenn $R_1$ für Wasserstoff steht;
$R_2$ Fluor, Methyl, Methoxy oder Methylthio bedeutet, wenn $R_1$ für Fluor steht; und

$R_2$ Chlor, Methyl, Methoxy oder Methylthio bedeutet, wenn $R_1$ für Chlor steht.

Wirkungsmässig hervorzuheben sind vor allem solche Verbindungen der Formel I, worin $R_1$ und $R_2$ gleichzeitig Fluor, Chlor und unabhängig voneinander Methyl, Methoxy oder Methylthio bedeuten, und insbesondere solche, worin $R_1$ und $R_2$ gleichzeitig Fluor oder Chlor bedeuten, vorzugsweise Fluor.

Weiterhin bevorzugte Verbindungen der Formel I sind dadurch gekennzeichnet, dass dass $R_3$ einen der Reste $-CHF_2$, $-CF_3$, $-CF_2CHF_2$, $-CH_2CF_3$, $-CF_2CHClF$, $-CF_2CHCl_2$, $-CF_2CCl_3$, $-CF_2CHBr_2$, $-CF_2CHBrF$, $-CF_2CHBr_2$, $-CH_2CHBrCH_2Br$, $-CF(CF_3)CHFCF_3$, $-CF_2CHFCF(CF_3)_2$, $-CF_2CHF(CF_2)_4CF_3$ oder

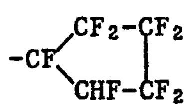

bedeutet,

insbesondere dadurch gekennzeichnet, dass $R_3$ einen der Reste $-CF_2CHF_2$, $-CF_2CHFCl$, $-CF_2CHCl_2$, oder $-CF_2CCl_3$ bedeutet. Interessant sind auch solche Verbindungen der Formel I, worin $R_3$ $C_5$-$C_7$-Halogenalkyl mit 1 bis 15 Halogenatomen bedeutet.

Die Verbindungen der Formel I können analog an sich bekannter Verfahren hergestellt werden (vgl. z.B. die deutschen Offenlegungsschriften Nr. 2.123.236, 2.601.780 und 3.240.975).

So kann man z.B. eine Verbindung der Formel I erhalten, durch Umsetzung

a) einer Verbindung der Formel II

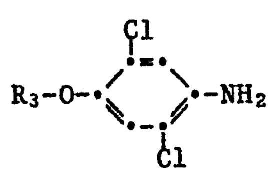

(II)

mit einer Verbindung der Formel III

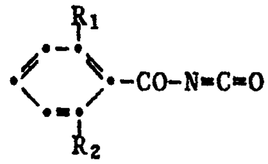

(III)

oder

b) einer Verbindung der Formel IV

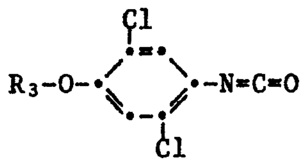

(IV)

mit einer Verbindung der Formel V

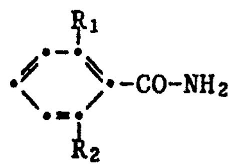

(V),

oder

c) einer Veibindung der Formel II mit einer Verbindung der Formel VI

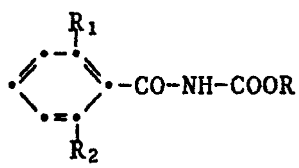

(VI).

In den obigen Formeln II bis VI haben die Reste $R_1$, $R_2$ und $R_3$ die vorstehend angegebenen Bedeutungen, und R bedeutet einen $C_1$-$C_8$-Alkylrest, der gegebenenfalls mit Halogen, vorzugsweise Chlor, substituiert ist.

Die erwähnten Verfahren a), b) und c) können vorzugsweise unter normalem Druck und in Gegenwart eines organischen Lösungs- oder Verdünnungsmittels durchgeführt werden. Als Lösungs- oder Verdünnungsmittel eignen sich z.B. Aether und ätherartige Verbindungen, wie Diäthyläther, Dipropyläther, Dibutyläther, Dioxan, Dimethoxy-

0179021

äthan und Tetrahydrofuran; N,N-dialkylierte Carbonsäureamide; aliphatische, aromatische sowie halogenierte Kohlenwasserstoffe, insbesondere Benzol, Toluol, Xylol, Chloroform, Methylenchlorid, Tetrachlorkohlenstoff und Chlorbenzol; Nitrile, wie Acetonitril oder Propionitril; Dimethylsulfoxid sowie Ketone, z.B. Aceton, Methyläthylketon, Methylisopropylketon und Methylisobutylketon. Verfahren a) wird im allgemeinen bei einer Temperatur von -10 bis 200°C, vorzugsweise zwischen 0 und 100°C, z.B. bei Raumtemperatur, gegebenenfalls in Gegenwart einer organischen Base, z.B. Triäthylamin, durchgeführt. Die Durchführung von Verfahren b) erfolgt bei einer Temperatur von 0 bis 150°C, vorzugsweise beim Siedepunkt des verwendeten Lösungsmittels, und gegebenenfalls in Gegenwart einer organischen Base, wie Pyridin, und/oder unter Zusatz eines Alkali-oder Erdalkalimetalls, vorzugsweise Natrium. Für das Verfahren c), d.h. für die Umsetzung der Urethane der Formel VI mit einem Anilin der Formel II, werden Temperaturen zwischen etwa 60°C und dem Siedepunkt des jeweiligen Reaktionsgemisches bevorzugt, wobei als Lösungsmittel insbesondere aromatische Kohlenwasserstoffe, wie Toluol, Xylole, Chlorbenzol usw., verwendet werden.

Die Ausgangsstoffe der Formeln III und V sind bekannt und können analog bekannten Verfahren hergestellt werden. Bei den Ausgangsstoffen der Formel II handelt es sich um neuartige Verbindungen, die ebenfalls einen Gegenstand der vorliegenden Erfindung bilden. Die Verbindungen der Formel II können in an sich bekannter Weise dadurch hergestellt werden, dass man entsprechend substituierte Nitrobenzole der Formel VII

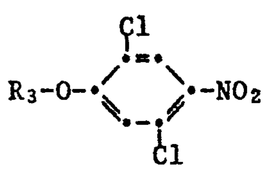

(VII)

in Analogie zu dem in J. Org. Chem. 29 (1964), 1, aufgezeigten Verfahren hydriert (vgl. auch die dort zitierte Literatur). Aber auch durch chemische Reduktion (z.B. mittels Sn-(II)-Chlorid/HCl) der entsprechenden Nitroverbindungen der Formel VII sind Aniline der

Formel II zugänglich (Vgl. Houben Weyl, "Methoden d. org. Chemie" 11/1, 422). Die Nitroverbindungen der Formel VII sind herstellbar durch Haloalkylierung von 2,5-Dichlor-4-Nitrophenol. Eine weitere Herstellungsmöglichkeit für die Aniline der Formel II besteht darin, dass man das acylierte 2,5-Dichlor-4-hydroxyanilin haloalkyliert und dann die Acylgruppe entfernt, z.B. durch saure Hydrolyse.

Zu Benzoylisocyanaten gemäss Formel III kann man unter anderem wie folgt gelangen (vgl. J. Agr. Food Chem. 21, 348 und 993; 1973):

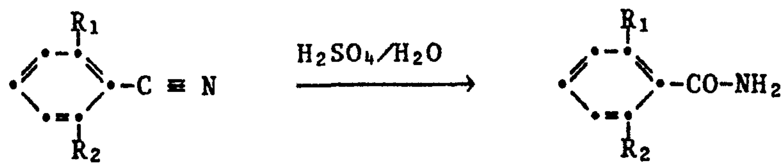


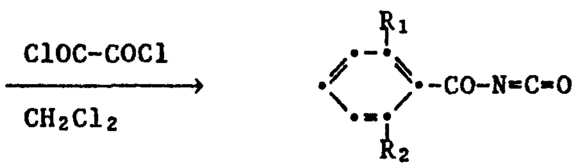


(III) .

Die 4-Haloalkoxy-phenylisocyanate der Formel IV lassen sich z.B. durch Phosgenierung der Aniline der Formel II nach allgemein üblichen Verfahren herstellen. Die weiterhin als Ausgangsstoffe zu verwendenden Benzamide der Formel V sind bekannt (vgl. z.B. Beilstein "Handbuch der organischen Chemie" Bd. 9, S. 336).

Urethane der Formel VI können in an sich bekannter Weise erhalten werden durch Umsetzung eines Benzoylisocyanats der Formel III mit einem entsprechenden Alkohol oder durch Umsetzung eines Benzamides der Formel V in Anwesenheit einer basischen Verbindung mit einem entsprechenden Ester der Chlorameisensäure Cl-COOR.

Ueberraschenderweise wurde gefunden, dass die vorliegenden Verbindungen der Formel I bei guter Pflanzenverträglichkeit und geringer Warmblütertoxizität ausgezeichnete Wirksamkeit als Schädlingsbe-

kämpfungsmittel aufweisen. Sie eignen sich vor allem zur Bekämpfung von Pflanzen und Tiere befallenden Insekten und Vertretern der Ordnung Akarina.

Insbesondere eignen sich die Verbindungen der Formel I zur Bekämpfung von Insekten der Ordnungen: Lepidoptera, Coleoptera, Homoptera, Heteroptera, Diptera, Thysanoptera, Orthoptera, Anoplura, Siphonaptera, Mallophaga, Thysanura, Isoptera, Psocoptera und Hymenoptera, sowie von Vertretern der Ordnung Akarina der Familien: Ixodidae, Argasidae, Tetranychidae und Dermanyssidae.

Neben ihrer Wirkung gegenüber Fliegen, wie z.B. Musca domestica, und Mückenlarven können Verbindungen der Formel I auch zur Bekämpfung von pflanzenschädigenden Frassinsekten in Zier- und Nutzpflanzungen, insbesondere in Baumwollkulturen (z.B. Spodoptera littoralis und Heliothis virescens), sowie in Obst- und Gemüsekulturen (z.B. Laspeyresia pomonella, Leptinotarsa decemlineata und Epilachna varivestis) eingesetzt werden. Die Verbindungen der Formel I zeichnen sich durch eine ausgeprägte ovizide und vor allem larvizide Wirkung gegen Insekten, insbesondere Larven von fressenden Schadinsekten, aus. Werden Verbindungen der Formel I von adulten Insekten-Stadien mit dem Futter aufgenommen, so ist in vielen Fällen, insbesondere bei Coleopteren, wie z.B. Anthonomus grandis, eine verminderte Ei-Ablage und/oder reduzierte Schlupfrate festzustellen.

Die Verbindungen der Formel I können ferner zur Bekämpfung von Ektoparasiten, wie Lucilia sericata, an Haus- und Nutztieren eingesetzt werden, z.B. durch Tier-, Stall- und Weidebehandlung.

Die Verbindungen der Formel I eignen sich ferner zur Bekämpfung der folgenden Obst- und Gemüsekulturen befallenden Milbenspezies: Tetranychus urticae, Tetranychus cinnabarinus, Panonychus ulmi, Broybia rubrioculus, Panonychus citri, Eriophyes piri, Eriophyes ribis, Eriophyes vitis, Tarsonemus pallidus, Phyllocoptes vitis und Phyllocoptruta oleivora.

Die gute pestizide Wirkung der erfindungsgemässen Verbindungen der Formel I entspricht einer Abtötungsrate (Mortalität) von mindestens 50-60 % der erwähnten Schädlinge.

Die Wirkung der erfindungsgemässen Verbindungen bzw. der sie enthaltenden Mittel lässt sich durch Zusatz von anderen Insektiziden und/oder Akariziden wesentlich verbreitern und an gegebene Umstände anpassen. Als Zusätze kommen z.B. Vertreter der folgenden Wirkstoffklassen in Betracht: Organische Phosphorverbindungen, Nitrophenole und Derivate, Formamidine, Harnstoffe, Carbamate, Pyrethroide, chlorierte Kohlenwasserstoffe und Bacillus thuringiensis-Präparate.

Die Verbindungen der Formel I werden in unveränderter Form oder vorzugsweise zusammen mit den in der Formulierungstechnik üblichen Hilfsmitteln eingesetzt und können daher z.B. zu Emulsionskonzentraten, direkt versprühbaren oder verdünnbaren Lösungen, verdünnten Emulsionen, Spritzpulvern, löslichen Pulvern, Stäubemitteln, Granulaten, auch Verkapselungen in z.B. polymeren Stoffen in bekannter Weise verarbeitet werden. Die Anwendungsverfahren, wie Versprühen, Vernebeln, Verstäuben, Verstreuen oder Giessen, werden ebenso wie die Mittel den angestrebten Zielen und den gegebenen Verhältnissen entsprechend gewählt.

Die Formulierung, d.h. die den Wirkstoff der Formel I, bzw. Kombinationen dieser Wirkstoffe mit anderen Insektiziden oder Akariziden, und gegebenenfalls einen festen oder flüssigen Zusatzstoff enthaltenden Mittel, Zubereitungen oder Zusammensetzungen, werden in bekannter Weise hergestellt, z.B. durch inniges Vermischen und/oder Vermahlen der Wirkstoffe mit Streckmitteln, wie z.B. mit Lösungsmitteln, festen Trägerstoffen, und gegebenenfalls oberflächenaktiven Verbindungen (Tensiden).

Als Lösungsmittel können in Frage kommen: Aromatische Kohlenwasserstoffe, bevorzugt die Fraktionen $C_8$ bis $C_{12}$, wie z.B. Xylolgemische oder substituierte Naphthaline, Phthalsäureester, wie Dibutyl- oder Dioctylphthalat, aliphatische Kohlenwasserstoffe, wie Cyclohexan,

Paraffine, Alkohole und Glykole sowie deren Aether und Ester, wie Aethanol, Aethylenglykol, Aethylenglykolmonomethyl- oder -äthyläther, Ketone, wie Cyclohexanon, stark polare Lösungsmittel, wie N-Methyl-2-pyrrolidon, Dimethylsulfoxid oder Dimethylformamid, sowie gegebenenfalls epoxidierte Pflanzenöle, wie epoxidiertes Kokosnussöl oder Sojaöl, oder Wasser.

Als feste Trägerstoffe, z.B. für Stäubemittel und dispergierbare Pulver, werden in der Regel natürliche Gesteinsmehle verwendet, wie Calcit, Talkum, Kaolin, Montmorillonit oder Attapulgit. Zur Verbesserung der physikalischen Eigenschaften können auch hochdisperse Kieselsäuren oder hochdisperse saugfähige Polymerisate zugesetzt werden. Als gekörnte, adsorptive Granulatträger kommen poröse Typen, wie z.B. Bimsstein, Ziegelbruch, Sepiolit oder Bentonit, als nicht sorptive Trägermaterialien z.B. Calcit oder Sand in Frage. Darüberhinaus kann eine Vielzahl von granulierten Materialien anorganischer oder organischer Natur, wie insbesondere Dolomit oder zerkleinerte Pflanzenrückstände, verwendet werden.

Als oberflächenaktive Verbindungen kommen je nach Art des zu formulierenden Wirkstoffes der Formel I oder der Kombinationen dieser Wirkstoffe mit andern Insektiziden oder Akariziden nichtionogene, kation- und/oder anionaktive Tenside mit guten Emulgier-, Dispergier- und Netzeigenschaften in Betracht. Unter Tensiden sind auch Tensidgemische zu verstehen.

Geeignete anionische Tenside können sowohl sog. wasserlösliche Seifen als auch wasserlösliche synthetische oberflächenaktive Verbindungen sein.

Als Seifen eignen sich die Alkali-, Erdalkali- oder gegebenenfalls substituierten Ammoniumsalze von höheren Fettsäuren ($C_{10}$-$C_{22}$), wie z.B. die Na- oder K-Salze der Oel- oder Stearinsäure, oder von natürlichen Fettsäuregemischen, die z.B. aus Kokosnuss- oder Tallöl

gewonnen werden können. Ferner sind als Tenside auch die Fettsäuremethyl-taurin-salze sowie modifizierte und nicht modifizierte Phospholipide zu erwähnen.

Häufiger werden jedoch sogenannte synthetische Tenside verwendet, insbesondere Fettsulfonate, Fettsulfate, sulfonierte Benzimidazolderivate oder Alkylarylsulfonate.

Die Fettsulfonate oder -sulfate liegen in der Regel als Alkali-, Erdalkali- oder gegebenenfalls substituierte Ammoniumsalze vor und weisen im allgemeinen einen Alkylrest mit 8 bis 22 C-Atomen auf, wobei Alkyl auch den Alkylteil von Acylresten einschliesst, z.B. das Na- oder Ca-Salz der Ligninsulfonsäure, des Dodecylschwefelsäureesters oder eines aus natürlichen Fettsäuren hergestellten Fettalkoholsulfatgemisches. Hierher gehören auch die Salze der Schwefelsäureester und Sulfonsäuren von Fettalkohol-Aethylenoxid-Addukten. Die sulfonierten Benzimidazolderivate enthalten vorzugsweise 2 Sulfonsäuregruppen und einen Fettsäurerest mit etwa 8-22 C-Atomen. Alkylarylsulfonate sind z.B. die Na-, Ca- oder Triäthanolaminsalze der Dodecylbenzolsulfonsäure, der Dibutylnaphthalinsulfonsäure oder eines Naphthalinsulfonsäure-Formaldehydkondensationsproduktes. Ferner kommen auch entsprechende Phosphate, wie z.B. Salze des Phosphorsäureesters eines p-Nonylphenol-(4-14)-Aethylenoxid-Adduktes, in Frage.

Als nichtionische Tenside kommen in erster Linie Polyglykolätherderivate von aliphatischen oder cycloaliphatischen Alkoholen, gesättigten oder ungesättigten Fettsäuren und Alkylphenolen in Frage, die 3 bis 30 Glykoläthergruppen und 8 bis 20 Kohlenstoffatome im (aliphatischen) Kohlenwasserstoffrest und 6 bis 18 Kohlenstoffatome im Alkylrest der Alkylphenole enthalten können. Weiterhin geeignete nichtionische Tenside sind die wasserlöslichen 20 bis 250 Aethylenglykoläthergruppen und 10 bis 100 Propylenglykoläthergruppen enthaltenden Polyäthylenoxid-Addukte an Polypropylenglykol, Aethylendiaminopolypropylenglykol und Alkylpolypropylenglykol mit

1 bis 10 Kohlenstoffatomen in der Alkylkette. Die genannten Verbindungen enthalten üblicherweise pro Propylenglykol-Einheit 1 bis 5 Aethylenglykoleinheiten.

Als Beispiele nichtionischer Tenside seien Nonylphenolpolyäthoxyäthanole, Ricinusölpolyglykoläther, Polypropylen-Polyäthylenoxid-Addukte, Tributylphenoxypolyäthoxyäthanol, Polyäthylenglykol und Octylphenoxypolyäthoxyäthanol erwähnt. Ferner kommen auch Fettsäureester von Polyoxyäthylensorbitan, wie das Polyoxyäthylensorbitantrioleat in Betracht.

Bei den kationischen Tensiden handelt es sich vor allem um quarternäre Ammoniumsalze, welche als N-Substituenten mindestens einen Alkylrest mit 8 bis 22 C-Atomen enthalten und als weitere Substituenten niedrige, gegebenenfalls halogenierte Alkyl-, Benzyl- oder niedrige Hydroxyalkylreste aufweisen. Die Salze liegen vorzugsweise als Halogenide, Methylsulfate oder Aethylsulfate vor, z.B. das Stearyltrimethylammoniumchlorid oder das Benzyl-di-(2-chloräthyl)-äthylammoniumbromid.

Die in der Formulierungstechnik gebräuchlichen Tenside sind u.a. in folgenden Publikationen beschrieben:

"Mc Cutcheon's Detergents and Emulsifiers Annual" MC Publishing Corp., Ridgewood, New Jersey, 1979;
Dr. Helmut Stache "Tensid Taschenbuch", Carl Hanser Verlag München/Wien 1981.

Die pestiziden Zubereitungen enthalten in der Regel 0,1 bis 99 %, insbesondere 0,1 bis 95 %, Wirkstoff der Formel I oder Kombinationen dieser Wirkstoffe mit andern Insektiziden oder Akariziden, 1 bis 99,9 % eines festen oder flüssigen Zusatzstoffes und 0 bis 25 %, insbesondere 0,1 bis 20 %, eines Tensides. Während als Handelsware eher konzentrierte Mittel bevorzugt werden, verwendet der Endverbraucher in der Regel verdünnte Zubereitungen, die wesentlich geringere Wirkstoffkonzentrationen aufweisen.

Die Mittel können auch weitere Zusätze wie Stabilisatoren, Entschäumer, Viskositätsregulatoren, Bindemittel, Haftmittel sowie Dünger oder andere Wirkstoffe zur Erzielung spezieller Effekte enthalten.

Beispiel 1:

a) Herstellung von 2,5-Dichlor-4-(1',1',2',2'-tetrafluoräthoxy)-anilin:

Es werden 18,6 g 4-Acetylamino-2,5-dichlorphenol zusammen mit 6,1 g KOH-Pulver und 130 ml Dimethylformamid in einem Autoklaven verrührt. In den geschlossenen Autoklaven werden dann 20 g Tetrafluoräthylen eingepresst, und das Gemisch wird während 20 Stunden bei 70°C unter dem sich im Autoklaven einstellenden Druck gerührt. Nach dem Abkühlen wird das Gemisch am Rotationsverdampfer eingeengt und der Rückstand in Methylenchlorid gelöst. Die erhaltene Lösung wird mit Wasser gewaschen, über $Na_2SO_4$ getrocknet und eingeengt. Nach dem Umkristallisieren aus einem Methylenchlorid/Hexan-Gemisch erhält man das 2,5-Dichlor-4-(1',1',2',2'-tetrafluoräthoxy)-acetanilid vom Smp. 119-120°C. 15,8 g des erhaltenen 2,5-Dichlor-4-(1',1',2',2'-tetrafluoräthoxy)-acetanilids werden mit 75 ml Aethanol und 25 ml 37%-iger Chlorwasserstoffsäure während 10 Stunden am Rückfluss gehalten. Das Reaktionsgemisch wird dann eingeengt, mit Eis-Wasser verdünnt und schwach alkalisch gestellt. Das Produkt wird aus dem Gemisch mit Methylenchlorid extrahiert. Die organische Phase wird mit Wasser gewaschen, über $Na_2SO_4$ getrocknet und das Methylenchlorid abdestilliert. Das Rohprodukt wird dann durch Vakuumdestillation gereinigt. Auf diese Weise erhält man die Titelverbindung der Formel

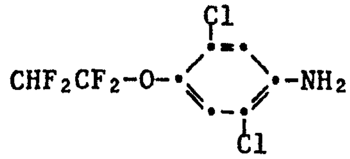

als farblose Flüssigkeit vom Siedepunkt 81-82°C/0.03 Torr.

Auf entsprechende Weise werden auch die folgenden Aniline der Formel II hergestellt:

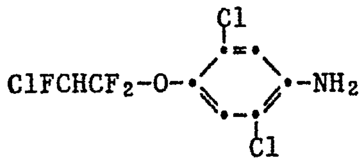

Kp.91-97°C/0.015 Torr

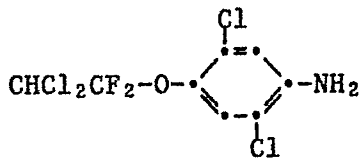

Kp.107-110°C/0.015 Torr

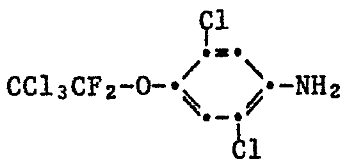

Kp.140°C/0.01 Torr

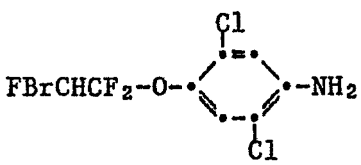

Kp.110-114°C/0.012 Torr

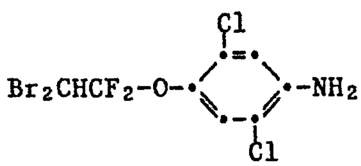

Smp.: 78°C

b) Herstellung von N-2-Chlorbenzoyl-N'-2,5-dichlor-4-(1',1',2',2'-tetrafluoräthoxy)-phenylharnstoff:

5,3 g 2,5-Dichlor-4-(1',1',2',2'-tetrafluoräthoxy)-anilin werden unter Rühren in 60 ml trockenem Toluol gelöst und bei Raumtemperatur unter Feuchtigkeitsausschluss mit 3,46 g 2-Chlorbenzoylisocyanat, gelöst in 10 ml trockenem Toluol, versetzt. Der Ansatz wird während 10 Stunden bei Raumtemperatur weitergerührt. Danach werden am Rotationsverdampfer etwa 75 Vol.-% des Lösungsmittels entfernt, der ausgefallene Rückstand wird abgesaugt, mit wenig kaltem Toluol und Hexan gewaschen und dann im Vakuum getrocknet.

0179021

Man erhält so die Titelverbindung der Formel

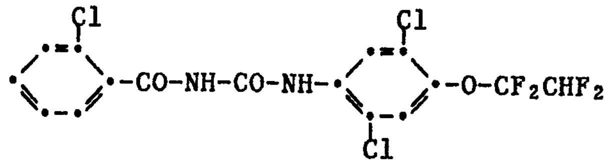

als farblose Kristalle mit einem Schmelzpunkt von 184-185°C (Verbindung Nr. 1).

In entsprechender Weise wie vorstehend beschrieben werden auch die folgenden Verbindungen der Formel I hergestellt:

| Verb. Nr. | $R_1$ | $R_2$ | $R_3$ | Smp.[°C] |
|---|---|---|---|---|
| 2 | H | F | $-CF_2CHF_2$ | 167-168 |
| 3 | F | $-OCH_3$ | $-CF_2CHF_2$ | 190-191 |
| 4 | H | Cl | $-CF_2CHFCl$ | 165-166 |
| 5 | F | Cl | $-CF_2CHFCl$ | 165-167 |
| 6 | H | Cl | $-CF_2CHCl_2$ | 165-167 |
| 7 | H | Cl | $-CF_2CHFBr$ | 161-162 |
| 8 | H | Cl | $-CF_2CHBr_2$ | 172 |
| 9 | F | Cl | $-CF_2CHBr_2$ | 188 |
| 10 | H | F | $-CF_2CHBr_2$ | 215 |
| 11 | F | $-OCH_3$ | $-CF_2CHBr_2$ | 202-203 |
| 12 | F | F | $-CF_2CHF_2$ | 183-184 |
| 13 | Cl | Cl | $-CF_2CHF_2$ | 211-213 |
| 14 | F | F | $-CF_2CHFCl$ | 174-175 |
| 15 | Cl | Cl | $-CF_2CHFCl$ | 202-207 |
| 16 | F | F | $-CF_2CHCl_2$ | 167-168 |
| 17 | F | F | $-CF_2CCl_3$ | 206-210 |
| 18 | F | F | $-CH_2CHBrCH_2Br$ | 190-192 |
| 19 | Cl | Cl | $-CH_2CHBrCH_2Br$ | 188-191 |
| 20 | F | F | $-CF_2CHFBr$ | 190 |
| 21 | F | F | $-CF_2CHBr_2$ | 185-186 |
| 22 | Cl | Cl | $-CF_2CHBr_2$ | 214-215 |
| 23 | F | F | $-CFClCHFCl$ | 148-150 |
| 24 | Cl | Cl | $-CFClCHFCl$ | 140-142 |
| 25 | F | Cl | $-CHF_2$ | 223-224 |
| 26 | F | F | $-CHF_2$ | 195-196 |
| 27 | H | Cl | $-CHF_2$ | 194 |

Ferner sind wie vorstehend beschrieben auch die folgenden Verbindungen der Formel I herstellbar:

| Verb. Nr. | $R_1$ | $R_2$ | $R_3$ |
|---|---|---|---|
| 28 | H | Cl | $-CF_2CHF_2$ |
| 29 | F | Cl | $-CF_2CHF_2$ |
| 30 | H | Cl | $-CF(CF_3)CHFCF_3$ |
| 31 | Cl | F | $-CF(CF_3)CHFCF_3$ |
| 32 | H | Cl | $-CF_2CHFCF(CF_3)_2$ |
| 33 | Cl | F | $-CF_2CHFCF(CF_3)_2$ |
| 34 | H | Cl | $-CF_2CHF(CF_2)_4CF_3$ |
| 35 | F | F | $-CF_3$ |
| 36 | $-CH_3$ | $-CH_3$ | $-CF_2CHF_2$ |
| 37 | $-OCH_3$ | $-OCH_3$ | $-CF_2CHF_2$ |
| 38 | $-SCH_3$ | $-SCH_3$ | $-CF_2CHF_2$ |
| 39 | Cl | Cl | $-CF_2CHF(CF_2)_4CF_3$ |
| 40 | H | Cl | 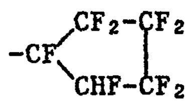 |
| 41 | Cl | F | 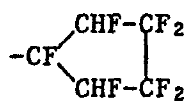 |

<u>Beispiel 2</u>:

<u>Formulierungen für Wirkstoffe der Formel I gemäss Beispiel 1 resp. Kombinationen dieser Wirkstoffe mit andern Insektiziden oder Akariziden (% = Gewichtsprozent)</u>

| 1. <u>Spritzpulver</u> | a) | b) | c) |
|---|---|---|---|
| Wirkstoff oder Wirkstoffkombination | 25 % | 50 % | 75 % |
| Na-Ligninsulfonat | 5 % | 5 % | - |
| Na-Laurylsulfat | 3 % | - | 5 % |
| Na-Diisobutylnaphthalinsulfonat | - | 6 % | 10 % |
| Octylphenolpolyäthylenglykoläther (7-8 Mol AeO) | - | 2 % | - |
| Hochdisperse Kieselsäure | 5 % | 10 % | 10 % |
| Kaolin | 62 % | 27 % | - |

Der Wirkstoff oder die Wirkstoffkombination wird mit den Zusatzstoffen gut vermischt und in einer geeigneten Mühle gut vermahlen. Man erhält Spritzpulver, die sich mit Wasser zu Suspensionen jeder gewünschten Konzentration verdünnen lassen.

2. <u>Emulsions-Konzentrat</u>

| | |
|---|---|
| Wirkstoff oder Wirkstoffkombination | 10 % |
| Octylphenolpolyäthylenglykoläther (4-5 Mol AeO) | 3 % |
| Ca-Dodecylbenzolsulfonat | 3 % |
| Ricinusölpolyglykoläther (36 Mol AeO) | 4 % |
| Cyclohexanon | 30 % |
| Xylolgemisch | 50 % |

Aus diesem Konzentrat können durch Verdünnen mit Wasser Emulsionen jeder gewünschten Konzentration hergestellt werden.

| 3. <u>Stäubemittel</u> | a) | b) |
|---|---|---|
| Wirkstoff oder Wirkstoffkombination | 5 % | 8 % |
| Talkum | 95 % | - |
| Kaolin | - | 92 % |

Man erhält anwendungsfertige Stäubemittel, indem der Wirkstoff oder die Wirkstoffkombination mit dem Träger vermischt und auf einer geeigneten Mühle vermahlen wird.

4. <u>Extruder-Granulat</u>

| | |
|---|---|
| Wirkstoff oder Wirkstoffkombination | 10 % |
| Na-Ligninsulfonat | 2 % |
| Carboxymethylcellulose | 1 % |
| Kaolin | 87 % |

Der Wirkstoff oder die Wirkstoffkombination wird mit den Zusatzstoffen vermischt, vermahlen und mit Wasser angefeuchtet. Dieses Gemisch wird extrudiert, granuliert und anschliessend im Luftstrom getrocknet.

5. Umhüllungs-Granulat

| | |
|---|---|
| Wirkstoff oder Wirkstoffkombination | 3 % |
| Polyäthylenglykol (MG 200) | 3 % |
| Kaolin | 94 % |

Der fein gemahlene Wirkstoff oder die Wirkstoffkombination wird in einem Mischer auf das mit Polyäthylenglykol angefeuchtete Kaolin gleichmässig aufgetragen. Auf diese Weise erhält man staubfreie Umhüllungs-Granulate.

6. Suspensions-Konzentrat

| | |
|---|---|
| Wirkstoff oder Wirkstoffkombination | 40 % |
| Aethylenglykol | 10 % |
| Nonylphenolpolyäthylenglykoläther (15 Mol AeO) | 6 % |
| Na-Ligninsulfonat | 10 % |
| Carboxymethylcellulose | 1 % |
| 37%ige wässrige Formaldehyd-Lösung | 0,2 % |
| Silikonöl in Form einer 75%igen wässrigen Emulsion | 0,8 % |
| Wasser | 32 % |

Der fein gemahlene Wirkstoff oder die Wirkstoffkombination wird mit den Zusatzstoffen innig vermischt. Man erhält so ein Suspensions-Konzentrat, aus welchem durch Verdünnen mit Wasser Suspensionen jeder gewünschten Konzentration hergestellt werden können.

Beispiel 3: Wirkung gegen Musca domestica:

Je 50 g frisch zubereitetes Nährsubstrat für Maden werden in Becher eingewogen. Von einer 1 Gew.-%igen acetonischen Lösung des betreffenden Wirkstoffes wird eine bestimmte Menge auf das in den Bechern befindliche Nährsubstrat pipettiert, so dass sich eine Wirkstoff-konzentration von 800 ppm ergibt. Nach dem Durchmischen des Substrates lässt man das Aceton mindestens 20 Stunden lang verdampfen.

Dann werden pro Wirkstoff und Konzentration je 25 eintägige Maden von Musca domestica in die das so behandelte Nährsubstrat enthaltenden Becher gegeben. Nachdem sich die Maden verpuppt haben, werden

die gebildeten Puppen durch Ausschwemmen mit Wasser von dem Substrat abgetrennt und in mit Siebdeckeln verschlossenen Gefässen deponiert. Die pro Ansatz ausgeschwemmten Puppen werden gezählt (toxischer Einfluss des Wirkstoffes auf die Madenentwicklung). Dann wird nach 10 Tagen die Anzahl der aus den Puppen geschlüpften Fliegen bestimmt.

Verbindungen der Formel I gemäss Beispiel 1 zeigen gute Wirkung im obigen Test.

Beispiel 4: Wirkung gegen Lucilia sericata:
Zu 9 ml eines Zuchtmediums wird bei 50°C 1 ml einer 0,5 Gew.-% Aktivsubstanz enthaltenden wässrigen Zubereitung hinzugefügt. Nun werden ca. 30 frisch geschlüpfte Lucilia sericata-Larven zum Zuchtmedium gegeben, und nach 48 und 96 Stunden wird die insektizide Wirkung durch Ermittlung der Abtötungsrate festgestellt.

Verbindungen der Formel I gemäss Beispiel 1 zeigen in diesem Test gute Wirkung gegen Lucilia sericata.

Beispiel 5: Wirkung gegen Aëdes aegypti:
Auf die Oberfläche von 150 ml Wasser, das sich in einem Behälter befindet, wird so viel einer 0,1 Gew.-%igen acetonischen Lösung des Wirkstoffes pipettiert, dass eine Konzentration von 800 ppm erhalten wird. Nach Verdunsten des Acetons wird der Behälter mit 30 bis 40 2-tägigen Aëdes-Larven beschickt. Nach 1, 2 und 5 Tagen wird die Mortalität geprüft.

Verbindungen der Formel I gemäss Beispiel 1 zeigen in diesem Test gute Wirkung gegen Aëdes aegypti.

Beispiel 6: Insektizide Frassgift-Wirkung:
Ca. 25 cm hohe eingetopfte Baumwollpflanzen werden mit wässrigen Wirkstoffemulsionen besprüht, die den Wirkstoff in einer Konzentration von 400 ppm enthalten.

Nach dem Antrocknen des Sprühbelages werden die Baumwollpflanzen mit Spodoptera littoralis- bzw. Heliothis virescens-Larven im dritten larvalen Stadium besiedelt. Der Versuch wird bei 24°C und 60 % relativer Luftfeuchtigkeit durchgeführt. Nach 120 Stunden wird die %-Mortalität der Test-Insekten bestimmt.

Eine Wirkung von 80-100 % (Mortalität) zeigt Verbindung Nr. 1 bei 400 ppm gegen Spodoptera-Larven und Heliothis-Larven.

Beispiel 7: Wirkung gegen Epilachna varivestis:
Etwa 15-20 cm hohe Phaseolus vulgaris-Pflanzen (Buschbohnen) werden mit wässrigen, den zu prüfenden Wirkstoff in einer Konzentration von 800 ppm enthaltenden Emulsions-Zubereitungen besprüht. Nach dem Antrocknen des Sprühbelages werden pro Pflanze 5 Larven von Epilachna varivestis (Mexikanischer Bohnenkäfer) im 4. larvalen Stadium angesetzt. Ueber die infestierten Pflanzen wird ein Plastikzylinder gestülpt, der mit einem Kupfer-Gazedeckel abgedeckt ist. Der Versuch wird bei 28°C und 60 % relativer Luftfeuchtigkeit durchgeführt.

Nach 2 und 3 Tagen wird die % Mortalität bestimmt. Zur Auswertung hinsichtlich allfälligem Frass-Schaden (Antifeeding-Effekt), Entwicklungs- und Häutungsstörungen werden die Versuchstiere während weiterer 3 Tage beobachtet.

Verbindungen der Formel I gemäss Beispiel 1 zeigen gute Wirkung im obigen Test.

Beispiel 8: Ovizide Wirkung auf Heliothis virescens und Spodoptera littoralis:
Entsprechende Mengenanteile einer benetzbaren pulverförmigen Formulierung, enthaltend 25 Gew.% des zu prüfenden Wirkstoffes, werden mit jeweils soviel Wasser vermischt, dass sich eine wässrige Emulsion mit einer Wirkstoffkonzentration von 400 ppm ergibt.

In diese wirkstoffhaltige Emulsion werden eintägige auf Cellophan abgelegte Eigelege von Heliothis und auf Papier abgelegte Eigelege von Spodoptera während drei Minuten eingetaucht und dann auf Rundfilter abgenutscht. Die so behandelten Gelege werden in Petrischalen ausgelegt und bei 28°C und 60 % relativer Luftfeuchtigkeit in der Dunkelheit aufbewahrt. Nach 5 bis 8 Tagen wird die Schlupfrate, d.h. die Anzahl Larven, die sich aus den behandelten Eiern entwickelt haben, gegenüber unbehandelten Kontrollansätzen bestimmt.

Verbindung Nr. 1 gemäss Beispiel 1 zeigt in obigem Test eine 80-100 %ige ovizide Wirkung (Mortalität) gegen Heliothis virescens und Spodoptera littoralis.

<u>Beispiel 9</u>: <u>Wirkung auf Laspeyresia pomonella (Eier)</u>:
Abgelegte Eier von Laspeyresia pomonella, die nicht älter als 24 Stunden sind, werden auf Filterpapier für 1 Minute in eine acetonisch-wässrige Lösung, enthaltend 800 ppm des zu prüfenden Wirkstoffes, eingetaucht. Nach dem Eintrocknen der Lösung werden die Eier in Petrischalen ausgelegt und bei einer Temperatur von 28°C belassen. Nach 6 Tagen wird der prozentuale Schlupf aus den behandelten Eiern bewertet und die %-Mortalität bestimmt.

Verbindungen der Formel I gemäss Beispiel 1 zeigen gute Wirkung in obigem Test.

<u>Beispiel 10</u>: <u>Reproduktions-Beeinflussung von Anthonomus grandis</u>:
Adulte Anthonomus grandis, die nach dem Schlupf nicht älter als 24 Stunden waren, werden in Gruppen zu jeweils 25 Käfern in Käfige mit Gitterwänden überführt. Die mit den Käfern besetzten Käfige werden sodann während 5 bis 10 Sekunden in eine acetonische Lösung, enthaltend 0,1 Gew.% des zu prüfenden Wirkstoffes, eingetaucht. Nachdem die Käfer wieder trocken sind, werden sie zur Kopulation und Eiablage in abgedeckte und Futter enthaltende Schalen eingesetzt. Abgelegte Eier werden zwei- bis dreimal wöchentlich mit fliessendem Wasser ausgeschwemmt, gezählt, durch zwei- bis dreistündiges

Einlegen in ein wässriges Desinfektionsmittel desinfiziert und dann in Schalen, die eine geeignete Larvaldiät enthalten, deponiert. Nach 7 Tagen wird untersucht, ob sich aus den deponierten Eiern Larven entwickelt haben.

Zur Ermittlung der Dauer des die Reproduktion beeinflussenden Effektes der zu prüfenden Wirkstoffe wird die Eiablage der Käfer während eines Zeitraums von etwa 4 Wochen überprüft. Die Bonitierung erfolgt anhand der Verminderung der Anzahl abgelegter Eier und der daraus geschlüpften Larven im Vergleich zu unbehandelten Kontrollen.

Verbindungen der Formel I gemäss Beispiel 1 zeigen eine gute reproduktionsreduzierende Wirkung im obigen Test.

Beispiel 11: Wirkung gegen Anthonomus grandis (Adulte)

Zwei eingetopfte Baumwollpflanzen im 6-Blattstadium werden jeweils mit einer wässrigen benetzungsfähigen Emulsions-Zubereitung enthaltend 400 ppm des zu prüfenden Wirkstoffes besprüht. Nach dem Antrocknen des Spritzbelages (nach etwa 1,5 Stunden) wird jede Pflanze mit 10 adulten Käfern (Anthonomus grandis) besiedelt. Plastikzylinder, deren obere Oeffnungen mit Gaze abgedeckt sind, werden dann über die behandelten, mit den Testtieren besiedelten Pflanzen gestülpt, um ein Abwandern der Käfer zu verhindern. Die so behandelten Pflanzen werden bei 25°C und etwa 60 % relativer Luftfeuchtigkeit gehalten. Die Auswertung erfolgt nach 2, 3, 4 und 5 Tagen unter Zugrundelegung der prozentualen Mortalität der eingesetzten Testtiere (% Rückenlage) sowie der Antifeedant-Wirkung gegenüber unbehandelten Kontrollansätzen.

Verbindung Nr. 1 gemäss Beispiel 1 zeigt in obigem Test eine 80 - 100 %-ige Wirkung (Mortalität).

Patentansprüche

1. Verbindung der Formel I

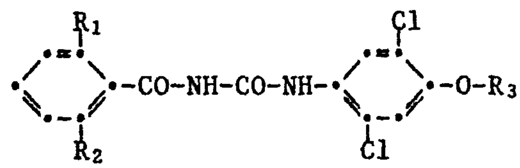

(I),

worin $R_1$ Wasserstoff, Fluor, Chlor, Methyl, Methoxy oder Methylthio, $R_2$ Fluor, Chlor, Methyl, Methoxy oder Methylthio und $R_3$ $C_1$-$C_7$-Halogenalkyl mit 1 bis 15 Halogenatomen oder $C_3$-$C_7$-Halogencycloalkyl mit 1 bis 13 Halogenatomen bedeuten, mit der Massgabe, dass $R_3$ nicht die Bedeutung $-CF_2-CHF-CF_3$ hat.

2. Verbindung der Formel I gemäss Anspruch 1, dadurch gekennzeichnet, dass $R_3$ mit 1 bis 7 Halogenatomen substituiertes $C_1$-$C_3$-Alkyl bedeutet.

3. Verbindung gemäss Anspruch 1 oder 2, dadurch gekennzeichnet, dass $R_2$ Methyl, Methoxy oder Methylthio bedeutet, wenn $R_1$ für Wasserstoff steht;
$R_2$ Fluor, Methyl, Methoxy oder Methylthio bedeutet, wenn $R_1$ für Fluor steht; und
$R_2$ Chlor, Methyl, Methoxy oder Methylthio bedeutet, wenn $R_1$ für Chlor steht.

4. Verbindung der Formel I gemäss Anspruch 3, dadurch gekennzeichnet, dass $R_1$ und $R_2$ gleichzeitig Fluor oder Chlor und unabhängig voneinander Methyl, Methoxy oder Methylthio bedeuten.

5. Verbindung der Formel I gemäss Anspruch 4, dadurch gekennzeichnet, dass $R_1$ und $R_2$ gleichzeitig Fluor oder Chlor bedeuten.

6. Verbindung der Formel I gemäss Anspruch 5, dadurch gekennzeichnet, dass $R_1$ und $R_2$ Fluor bedeuten.

7. Verbindung der Formel I gemäss einem der Ansprüche 1 bis 6 dadurch gekennzeichnet, dass $R_3$ einen der Reste $-CHF_2$, $-CF_3$, $-CF_2CHF_2$, $-CH_2-CF_3$, $-CF_2CHClF$, $-CF_2CHCl_2$, $-CF_2CCl_3$, $-CF_2CHBr_2$, $-CF_2CHBrF$, $-CF_2CHBr_2$, $-CH_2CHBrCH_2Br$, $-CF(CF_3)CHFCF_3$, $-CF_2CHFCF(CF_3)_2$, $-CF_2CHF(CF_2)_4CF_3$ oder

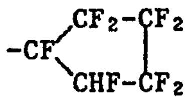

bedeutet.

8. Verbindung der Formel I gemäss Anspruch 7, dadurch gekennzeichnet, dass $R_3$ einen der Reste $-CF_2CHF_2$, $-CF_2CHFCl$, $-CF_2CHCl_2$, oder $-CF_2CCl_3$ bedeutet.

9. Verbindungen der Formel I gemäss Anspruch 8, dadurch gekennzeichnet, dass $R_3$ $C_5$-$C_7$-Halogenalkyl mit 1 bis 15 Halogenatomen bedeutet.

10. Verbindung gemäss Anspruch 6 der Formel

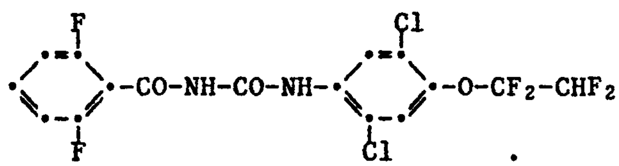

.

11. Verbindung gemäss Anspruch 6 der Formel

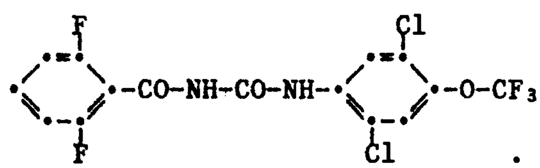

.

12. Verbindung gemäss Anspruch 6 der Formel

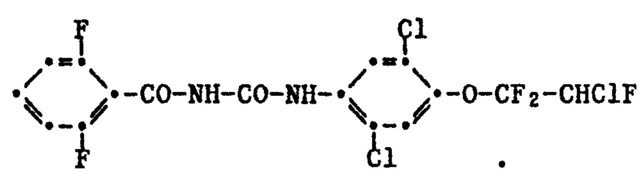

.

13. Verbindung gemäss Anspruch 6 der Formel

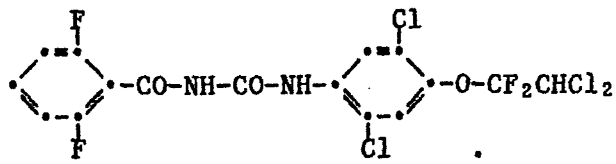

.

14. Verbindung gemäss Anspruch 6 der Formel

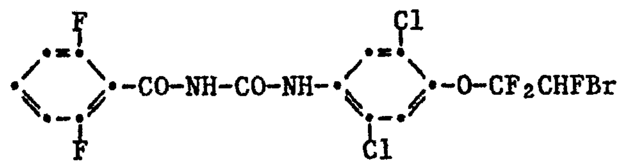

.

15. Verbindung gemäss Anspruch 2 der Formel

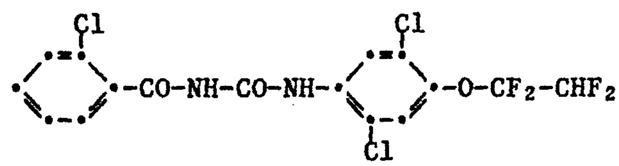

.

16. Verbindung gemäss Anspruch 2 der Formel

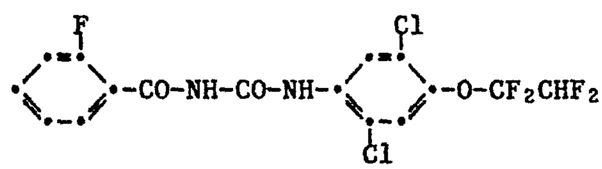

.

17. Verbindung gemäss Anspruch 3 der Formel

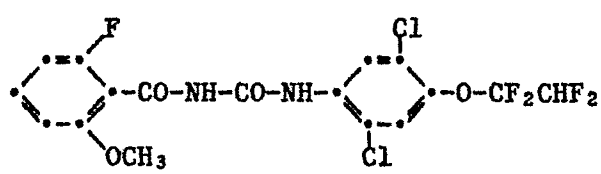

.

18. Verbindung gemäss Anspruch 3 der Formel

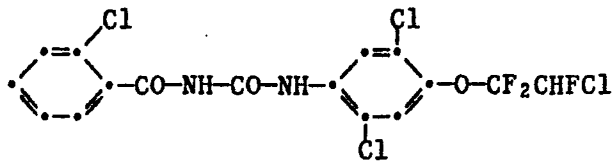

.

19. Verbindung gemäss Anspruch 2 der Formel

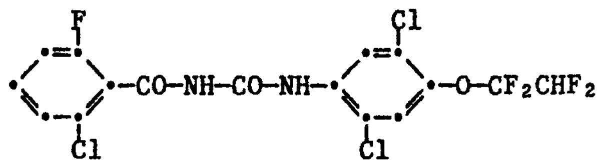

.

20. Verbindung gemäss Anspruch 3 der Formel

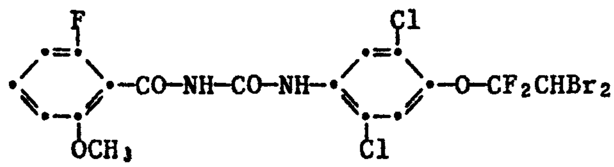

.

21. Verfahren zur Herstellung einer Verbindung gemäss einem der Ansprüche 1 bis 20, dadurch gekennzeichnet, dass man

a) eine Verbindung der Formel II

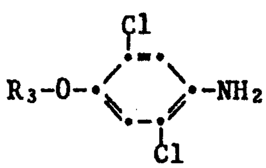

(II)

mit einer Verbindung der Formel III

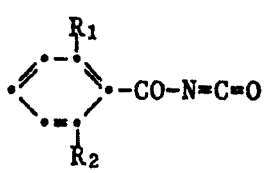

(III)

oder

b) eine Verbindung der Formel IV

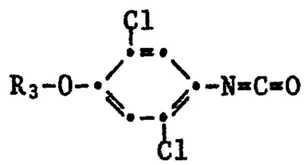

(IV)

mit einer Verbindung der Formel V

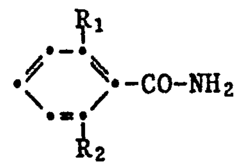

(V),

oder

c) eine Verbindung der Formel II mit einer Verbindung der Formel VI

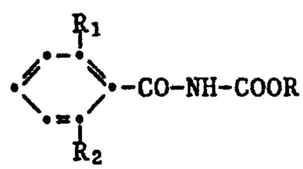

(VI)

umsetzt, wobei in den Formeln II bis VI die Reste $R_1$, $R_2$ und $R_3$ die in den Ansprüchen 1 bis 9 angegebenen Bedeutungen haben und R für einen $C_1$-$C_8$-Alkylrest, der gegebenenfalls mit Halogen substituiert ist, steht.

22. Verbindung der Formel II

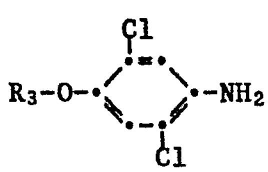

(II) ,

worin $R_3$ die unter den Ansprüchen 1 bis 9 angegebenen Bedeutungen hat.

23. Verbindung der Formel IV

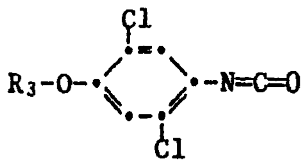

(IV) ,

worin $R_3$ die unter den Ansprüchen 1 bis 9 angegebenen Bedeutungen hat.

24. Schädlingsbekämpfungsmittel, welches als aktive Komponente eine Verbindung gemäss einem der Ansprüche 1 bis 20 zusammen mit geeigneten Trägern und/oder anderen Zuschlagstoffen enthält.

25. Verwendung einer Verbindung gemäss einem der Ansprüche 1 bis 20 zur Bekämpfung von Insekten und Vertretern der Ordnung Akarina.

26. Verwendung gemäss Anspruch 25 zur Bekämpfung larvaler Stadien pflanzenschädigender Insekten.

27. Verwendung gemäss Anspruch 25 als Insekten-Ovizid.

28. Verfahren zur Bekämpfung von Insekten und Vertretern der Ordnung Akarina, dadurch gekennzeichnet, dass man diese Schädlinge bzw. deren verschiedene Entwicklungsstadien oder deren Aufenthaltsort mit einer pestizid wirksamen Menge einer Verbindung der Formel I gemäss einem der Ansprüche 1 bis 20 oder mit einem Mittel enthaltend neben Zusatz- und Trägerstoffen eine pestizid wirksame Menge dieser Verbindung, in Kontakt bringt oder behandelt.

FO 7.5/EIC/eg*